# EUROPÄISCHE PATENTSCHRIFT

(11) **EP 0 973 035 B1**
(45) Veröffentlichungstag und Bekanntmachung des Hinweises auf die Patenterteilung: **25.08.2004**
(21) Anmeldenummer: 99112194.8
(22) Anmeldetag: 24.06.1999
(51) Int. Cl.: G01N 33/58, G01N 33/532, C12Q 1/68, C07F 19/00

(54) **Verwendung derivatisierter Reagenzien in Chemilumineszenz- und Elektrochemilumineszenz-Nachweisverfahren**
Use of derivatized reagents in chemiluminescence and electrochemiluminescence detection procedures
Utilisation d'agents dérivés en procédés de décèlement par chimioluminescence et electrochimioluminescence

(30) Priorität: 25.06.1998 DE 19828441
(43) Veröffentlichungstag der Anmeldung: 19.01.2000
(73) Patentinhaber: Roche Diagnostics GmbH, 68305 Mannheim (DE)
(72) Erfinder: Egger, Martin, 82347 Bernried (DE); Josel, Hans-Peter, 82362 Weilheim (DE); Punzmann, Gabriele, 81547 München (DE)
(74) Vertreter: Weiss, Wolfgang, Dipl.-Chem. Dr.

(56) Entgegenhaltungen:
- EP-A- 0 178 450
- EP-A- 0 580 979
- WO-A-87/06706
- WO-A-90/05296
- WO-A-96/03409
- WO-A-96/03410
- WO-A-96/35812
- US-A- 5 310 687

## Beschreibung

Die Erfindung betrifft Verfahren zum Nachweis eines Analyten in einer Probe durch Chemilumineszenz oder Elektrochemilumineszenz unter Verwendung derivatisierter Testreagenzien. Weiterhin werden neue Reagenzien und Reagenzienkits für Chemilumineszenz- und Elektrochemilumineszenz-Nachweisverfahren offenbart.

Der Nachweis von Analyten durch Chemilumineszenz und Elektrochemilumineszenz ist bekannt. So offenbart EP-A-0 178 450 Konjugate von Rutheniumkomplexen und immunologisch aktiven Materialien, die als Reagenzien in Chemilumineszenz-Nachweisverfahren eingesetzt werden. Die Rutheniumkomplexe enthalten drei gleiche oder verschiedene bi- oder polyzyklische Liganden mit mindestens zwei stickstoffhaltigen Heterozyklen, wobei mindestens einer dieser Liganden mit mindestens einer wasserlöslich machenden Gruppe wie -SO₃H oder -COOH substituiert ist und wobei mindestens einer dieser Liganden mit mindestens einer reaktiven Gruppe wie - COOH direkt oder über eine Spacergruppe substituiert ist und wobei die Liganden über Stickstoffatome an das Ruthenium gebunden sind.

Weiterhin ist die Verwendung von Metallkomplexen als Markierungsreagenzien für ein Elektrochemilumineszenz-Nachweisverfahren bekannt (vgl. z.B. EP-A-0 580 979, WO 87/06706, US 5,238,108 oder US 5,310,687). Ein solches Chemilumineszenz- oder Elektrochemilumineszenz-Nachweisverfahren beruht darauf, daß das Zentralatom des Metallkomplexes, z.B. Ruthenium, durch chemische oder elektrochemische Mittel in einer geeigneten Meßvorrichtung über einen energieverbrauchenden Prozeß, z.B Elektronentransfer aus einem Cosubstrat wie etwa einem Tripropylaminradikal in den angeregten MLCT-Triplettzustand überführt wird. Von diesem angeregten Zustand kann es unter Emission eines Photons durch einen verbotenen Triplett-Singulett-Übergang in den Grundzustand relaxieren (vgl. z.B. WO 90/05296; Leland und Powell, J. Electrochem. Soc. 137 (1990), 3127-3131; Blackburn et al., Clin. Chem. 37 (1991), 1534-1539). Dieser Reaktionsmechanismus unterliegt jedoch in Abhängigkeit der Bedingungen vor und während der Meßphase einer Vielzahl von Störungen. So können Nebenreaktionen die Lichtausbeute bis hin zum vollständigen Löschen vermindern.

Überraschenderweise wurde im Rahmen der vorliegenden Erfindung erkannt, daß leicht oxidierbare chemische Gruppen in Komponenten des Testreagenz, z.B. freie Aminogruppen, eine Störung der Lumineszenzreaktion bewirken können. Von den Erfindern durchgeführten Versuche zeigen, daß die störenden Gruppen dabei auf dem die Chemilumineszenz-Markierungsgruppe tragenden Molekül oder auf separaten Molekülen vorliegen können.

Von den Erfindern wurde erkannt, daß diese oxidierbaren, die Chemilumineszenz-Nachweisreaktion störenden Gruppen durch chemische Derivatisierung (Capping) blockiert und für eine chemisch oder elektrochemische Reaktion unzugänglich gemacht werden können, ohne daß gleichzeitig in einem Nachweisverfahren unzumutbare Sensitivitätseinbußen auftreten. Überraschend und neu ist insbesondere der experimentelle Befund, daß derivatisierte Verbindungen, insbesondere solche, die eine größere Anzahl von Chemilumineszenz-Markierungsgruppen tragen, mehr Licht emittieren als entsprechende underivatisierte Verbindungen und daß bei einem Elektrochemilumineszenz-Nachweisverfahren der katalytische anodische Stromanteil derivatisierter Verbindungen, der zu einer unerwünschten Verringerung des Meßsignals führt, bei derivatisierten Verbindungen kleiner als bei underivatisierten Verbindungen ist. Somit können durch Verwendung derivatisierter Testreagenzien in Chemilumineszenz- und Elektrochemilumineszenz-Nachweisverfahren signifikante Vorteile erzielt werden.

Ein Gegenstand der Erfindung ist somit ein Verfahren zum Nachweis eines Analyten in einer Probe durch Chemilumineszenz oder Elektrochemilumineszenz, wobei man die Probe mit einem Testreagenz in Kontakt bringt, das eine Chemilumineszenz- oder Elektrochemilumineszenz-Markierungsgruppe gekoppelt an einen analytspezifischen Rezeptor und weitere Testkomponenten enthält, und wobei das Verfahren dadurch gekennzeichnet ist, daß oxidierbare chemische Gruppen auf dem markierten Rezeptor oder/und weiteren Testkomponenten zumindest teilweise durch Derivatisierung geschützt werden.

Das erfindungsgemäße Verfahren kann ein Chemilumineszenz-Verfahren sein, d.h. ein von der Markierungsgruppe ausgehendes Signal wird durch chemische Mittel erzeugt. Vorzugsweise ist das Verfahren jedoch ein Elektrochemilumineszenz-Verfahren, bei dem das von der Markierungsgruppe ausgehende Lichtsignal durch elektrochemische Mittel erzeugt wird. Ein Elektrochemilumineszenz-Nachweisverfahren kann in bekannten Meßvorrichtungen durchgeführt werden, die beispielsweise eine Meßkammer zur Aufnahme einer Meßelektrode, Mittel zur Zufuhr und Ableitung von Flüssigkeiten aus der Meßkammer und Mittel zum Nachweis der aus der Meßkammer erzeugten Elektrochemilumineszenz aufweisen. Weiterhin kann die Vorrichtung vorzugsweise magnetische Mittel zur Immobilisierung magnetischer Partikel in der Probenflüssigkeit an der Meßelektrode aufweisen. Derartige Meßvorrichtungen sind beispielsweise von N. R. Hoyle: "The Application of Electrochemiluminescence to Immunoassay-based Analyte Measurement", in "Bioluminescence and Chemiluminescence"; Proceedings of the 8th International Symposium on Bioluminescence and Chemiluminescence, Cambridge, September 1994, A. K. Campbell et al., (Hrsg.), John Wiley & Sons sowie in WO89/10551 oder WO90/11511 beschrieben.

Das erfindungsgemäße Verfahren umfaßt die Verwendung von Testreagenzien, in denen oxidierbare chemische Gruppen, die die Chemilumineszenz oder Elektrochemilumineszenz-Nachweisreaktion stören, mindestens teilweise durch Derivatisierung geschützt, d.h. in unter den Testbedingungen nicht oder signifikant weniger oxidierbare Gruppen überführt worden sind. Die oxidierbaren chemischen Gruppen werden beispielsweise ausgewählt aus primären Aminogruppen, sekundären Aminogruppen, Schiffbasen, Thiolgruppen, Acetalgruppen und Ketalgruppen. Besonders bevorzugt sind primäre oder/und sekundäre aliphatische oder aromatische, insbesondere aliphatische Aminogruppen.

Die Derivatisierung der oxidierbaren Gruppen kann durch an sich bekannte Methoden erfolgen. Eine besonders bevorzugte Methode der Derivatisierung ist die die Acylierung insbesondere bei primären oder sekundären Aminogruppen. Andere Methoden der Derivatisierung beinhalten beispielsweise die Imidbildung, z. B. die Einführung einer Phthalimidgruppe (ausgehend von Phthalsäureanhydrid oder von O-Methoxycarbonyl-benzoylchlorid oder N-Ethoxycarbonylphthalimid), die Carbamatbildung (Carbamoylierung) und insbesondere die Sulfonylierung (z.B. die Einführung von Benzolsulfonamid-, bzw. p-Toluolsulfonamidgruppen). Weiterhin ist auch die Bildung quartärer Ammoniumsalze möglich.

Für die Acylierung können bekannte Acylierungsreagenzien wie etwa Carbonsäurechloride, -anhydride oder -aktivester verwendet werden. Bevorzugt sind Anhydride oder/und Aktivester. Besonders bevorzugt sind Aktivester wie etwa N-Hydroxysuccinimidester, vor allem lösliche Derivate davon, z.B. Sulfonsäurereste. Des weiteren kommen auch Essigsäure-pnitrobenzylester in Frage. Bei den zur Derivatisierung verwendeten Acylgruppen kann es sich vorzugsweise um kurzkettige Acylgruppen wie etwa Acetyl- oder und Propionylreste handen. Andererseits ist auch die Verwendung von hydrophile Gruppen, z.B. Hydroxyl-, Ether- oder Carbonsäuregruppen tragenden Acylresten wie etwa Tartryl- oder Succinylresten oder Polyethylenglykol-modifizierten Acylresten bevorzugt.

Die erfindungsgemäß derivatisierten Substanzen sind üblicherweise biologische Substanzen, wie sie als Testkomponenten in Nachweisverfahren zur Bestimmung von Analyten eingesetzt werden. Beispielsweise können die derivatisierten Substanzen ausgewählt werden aus Zellen, Viren, subzellulären Teilchen, Proteinen, Lipoproteinen, Glycoproteinen, Peptiden, Polypeptiden, Nukleinsäuren, Nukleinsäureanaloga, Oligosacchariden, Polysacchariden, Lipopolysacchariden, zellulären Metaboliten, Haptenen, Hormonen, pharmakologischen Wirkstoffen, Alkaloiden, Steroiden, Vitaminen und Aminosäuren. Vorzugsweise werden die derivatisierten Substanzen ausgewählt aus Peptiden, Polypeptiden, Nukleinsäuren, Nukleinsäureanaloga wie etwa peptidischen Nukleinsäuren (PNA), Zuckern und Haptenen, d.h. organischen Molekülen mit einem Molekulargewicht von 150 bis 2000. Besonders bevorzugt werden die derivatisierten Substanzen ausgewählt aus Polypeptiden wie etwa Antikörpern oder Antikörperfragmenten.

Als Chemilumineszenz-oder Elektrochemilumineszenz-Markierungsgruppen verwendet man vorzugsweise Metallkomplexe mit einer Struktur der allgemeinen Formel (I):

Mₙ(L₁L₂L₃)ₘ (I)

worin
- M: ein zwei- oder dreiwertiges Metallkation ausgewählt aus Seltenerdeund Übergangsmetallkationen wie etwa Ruthenium, Osmium, Rhenium, Iridium, Rhodium, Platin, Indium, Palladium, Molybdän, Techneticum, Kupfer, Chrom, Wolfram, Yttrium oder Luthetium ist,
- L₁, L₂ und L₃: gleich oder verschieden sind und Liganden mit mindestens 2 stickstoffhaltigen Heterocyclen, z.B. aromatische Heterocyclen wie Bipyridyl, Bipyrazyl, Terpyridyl oder Phenanthronyl sind und
- n und m: jeweils unabhängig ganze Zahlen von 1 bis 10, vorzugsweise von 1 bis 3 sind.

Besonders bevorzugte Metallkationen sind Ruthenium, Iridium, Rhenium, Chrom und Osmium. Am meisten bevorzugt ist Ruthenium. Zum Ladungsausgleich kann der Metallkomplex gegebenenfalls noch Gegenionen, z.B. Anionen, enthalten. Weiterhin ist bevorzugt, daß n und m jeweils 1 sind. Die Komplexliganden werden besonders bevorzugt aus Bipyridin- und Phenanthrolin-Ringsystemen ausgewählt.

Besonders gute Ergebnisse, insbesondere bei Elektrochemilumenszenz-Verfahren erhält man mit Metallkomplexen, die mindestens eine hydrophile Gruppe oder/und einen Ladungsträger (der vom Metallkation verschieden ist) im Komplex aufweisen. Beispiele solcher Ladungsträger sind Phosphat-, Phosphonat-, Sulfonat- und Carboxylatgruppen, wobei Sulfonat- und Carboxylatgruppen besonders bevorzugt sind. Beispiele für geeignete hydrophile Gruppen sind C₂-C₃-Alkylenoxy-Einheiten, C₂-C₃-Alkylenthio-Einheiten und Polyhydroxy-Einheiten. Derartige hydrophile oder/und geladene Metallkomplexe werden in EP-A-0 178 540, WO 96/03409 und WO96/03410 offenbart.

Die Herstellung solcher Metallkomplexe kann nach bekannten Methoden erfolgen, beispielsweise durch Reaktion eines Metallsalzes, z.B. eines Metallhalogenids, und gegebenenfalls anschließenden Austausch des Halogenidions durch Hexafluorphosphat-, Trifluoracetat- oder Tetrafluorboratgruppen. Der Metallkomplex wird anschließend nach bekannten Verfahren an biologische Substanz gekoppelt. Die resultierenden Konjugate können dann als Nachweisreagenzien eingesetzt werden.

Das erfindungsgemäße Verfahren umfaßt die Bestimmung eines Analyten in einer Probe. Die Probe ist vorzugsweise eine biologische Probe und liegt in flüssiger Form vor. Sie kann aus menschlichen, tierischen oder pflanzlichen Geweben, Körperflüssigkeiten, prokaryontischen oder eukaryontischen Zellkulturen etc. stammen. Dieser Probe werden die zur Bestimmung des jeweiligen Analyten benötigten Testreagenzien zugesetzt, die eine Chemilumineszenz- oder Elektrochemilumineszenz-Markierungsgruppe gekoppelt an eine biologische Substanz sowie weitere Testkomponenten, wie sie dem Fachmann bekannt sind, enthalten.

Das erfindungsgemäße Verfahren umfaßt die Bestimmung eines Analyten durch Reaktion mit einem oder mehreren analytspezifischen Rezeptoren aus dem Testreagenz. Diese Reaktion ist vorzugsweise eine hochaffine Wechselwirkung, z.B. eine Antigen-Antikörper-Reaktion, eine Nukleinsäure-Hybridisierungsreaktion oder/und eine Zucker-Lectin-Reaktion. Vorzugsweise ist das erfindungsgemäße Verfahren ein immunologisches Verfahren oder ein Nukleinsäure-Hybridisierungsverfahren.

Das Nachweisverfahren kann als homogener Assay, d.h. Messung der Chemilumineszenz oder Elektrochemilumineszenz in der Flüssigphase durchgeführt werden. Vorzugsweise wird jedoch ein heterogener Test durchgeführt, bei dem man die Chemilumineszenz- oder Elektrochemilumineszenzmarkierung an einer Festphase, z.B. einer partikulären Festphase wie etwa magnetischen Mikrobeads, z.B. Streptavidin-beschichteten Mikrobeads, oder kolloidalen Partikeln immobilisiert und das Vorhandensein oder/und die Menge des Analyten über die an der Festphase immobilisierte Markierung bestimmt. Bei Durchführung eines heterogenen Tests umfaßt das Verfahren sog. Einfang- und Waschschritte, bei denen die Festphase an der Elektrode immobilisiert wird und eine Abtrennung übriger nichtgebundener Probenbestandteile erfolgt.

Die Erfindung beruht auf der Verwendung von einer oder mehreren derivatisierten Testkomponenten bei der Bestimmung eines Analyten durch Chemilumineszenz oder Elektrochemilumineszenz. In einer besonders bevorzugten Ausführungsfom wird der die Chemilumineszenz- oder Elektrochemilumineszenz-Markierungsgruppe tragende analytspezifische Rezeptor in derivatisierter Form eingesetzt. Bei diesem Rezeptor handelt es sich um ein vorzugsweise kovalentes Konjugat aus einer biologischen Substanz - und einer oder mehreren Markierungsgruppen. Die biologische Substanz des Konjugats ist "analytspezifisch", d.h. sie kann eine spezifische Wechselwirkung direkt mit dem Analyten oder einem weiteren analytspezifischen Rezeptor eingehen (Sandwich-Test) oder es kann sich um ein Analytanalogon handeln (kompetitiver Test). Der analytspezifische Rezeptor kann eine oder mehrere Markierungsgruppen tragen. Besonders bevorzugt wird der analytspezifische Rezeptor in einer hochmarkierten Form eingesetzt, d.h. er trägt mindestens 5, besonders bevorzugt mindestens 10 Markierungsgruppen pro Molekül der biologischen Substanz.

Alternativ oder zusätzlich zur Derivatisierung des markierten analytspezifischen Rezeptors können auch weitere Testkomponenten in derivatisierter Form eingesetzt werden. Zu derartigen weiteren Testkomponenten zählen nichtmarkierte analytspezifische Rezeptoren, Festphasenbeschichtungen und - sofern vorhanden - Entstörungssubstanzen. Nichtmarkierte analytspezifische Rezeptoren sind beispielsweise Substanzen, die in einem Sandwichtest zur Immobilisierung des Analyten an einer Festphase benötigt werden, oder freie Rezeptoren, die direkt mit den Analyten wechselwirken können, und an die dann wiederum der markierte Rezeptor bindet. Bei Festphasenbeschichtungen kann es sich um Polypeptide wie etwa Antikörper oder Streptatvidin handeln. Falls Entstörungsreagenzien verwendet werden, beispielsweise unspezifische Antikörper, können auch diese in derivatisierter Form eingesetzt werden.

Die Derivatisierung von biologischen Substanzen kann statistisch, aber auch selektiv erfolgen. Eine statistische Derivatisierung oxidierbarer Gruppen wird häufig bei Makromolekülen wie etwa Polypeptiden, z.B. Antikörpern, mit Erfolg eingesetzt. Hierzu wird die biologische Substanz mit dem Derivatisierungsreagenz, z.B. einem Acylierungsreagenz, in geeigneter Menge unter geeigneten Bedingungen in Kontakt gebracht, um einen gewünschten Derivatisierungsgrad zu erreichen, der einerseits eine signifikante Verbesserung des Chemilumineszenz- oder Elektrochemilumineszenz-Meßsignals während des Nachweisverfahrens ermöglicht, aber andererseits nicht zu einer signifikanten Beeinträchtigung der Testperformance der biologischen Substanz, z.B. Analytspezifität, Entstörungskapazität etc. führt. Reaktionsbedingungen, diezu einemgeeigneten Derivatisierungsgrad der biologischen Substanz führen, können vom Fachmann auf einfache Weise ermittelt werden.

Andererseits kann auch eine selektive Derivatisierung von Testkomponenten erfolgen. Dabei werden gezielt bestimmte Gruppen der biolgischen Substanz gezielt derivatisiert. Diese Vorgehensweise kommt insbesondere bei kleineren biologischen Substanzen wie etwa Haptenen, Peptiden, aber auch bei Nukleinsäuren oder Nukleinsäureanaloga in Betracht. Gezielte Derivatisierungsreaktionen lassen sich beispielsweise bei chemischen Syntheseverfahren von Peptiden, Nukleinsäuren oder peptidischen Nukleinsäuren auf einfache Weise durch Verwendung geeigneter Schutzgruppenstrategien bzw. durch Einsatz von bereits derivatisierten Monomerbausteinen an jeweils gewünschten Positionen ausführen.

Ein weiterer Gegenstand der vorliegenden Erfindung ist ein Reagenzienkit zum Nachweis eines Analyten umfassend:
(a) einen analytspezifischen Rezeptor, der mindestens eine Chemilumineszenz- oder Elektrochemilumineszenz-Markierungsgruppe trägt, und
(b) weitere Testkomponenten,
dadurch gekennzeichnet, daß oxidierbare chemische Gruppen auf dem markierten Rezeptor oder/und auf weiteren Testkomponenten durch Derivatisierung geschützt sind.

Die weiteren Testkomponenten umfassen biologische Substanzen, die vorzugsweise aus nichtmarkierten analytspezifischen Rezeptoren, Festphasenbeschichtungen oder/und Entstörungssubstanzen ausgewählt sind. Eine oder mehrere Substanzen aus der Gruppe bestehend aus dem analytspezifischen Rezeptor und diesen weiteren Testkomponenten liegen in derivatisierter Form vor. Vorzugsweise ist mindestens der markierte analytspezifische Rezeptor derivatisiert. Der erfindungsgemäße Reagenzienkit kann in einem Verfahren zum Nachweis eines Analyten, insbesondere in einem der zuvor genannten Verfahren, eingesetzt werden.

Noch ein weiterer Gegenstand der Erfindung ist ein Konjugat einer biologischen Substanz und mindestens einer Chemilumineszenz- oder Elektrochemilumineszenz-Markierungsgruppe, welches dadurch gekennzeichnet ist, daß oxidierbare chemische Gruppen auf der biologischen Substanz durch Derivatisierung geschützt sind.

Weiterhin wird die Erfindung durch die nachfolgenden Beispiele und Abbildungen erläutert. Es zeigen:
- Abb. 1 und 2: die Ergebnisse von cyclovoltammetrischen Messungen an derivatisierten und nichtderivatisierten Rutheniummarkierten Antikörpern und

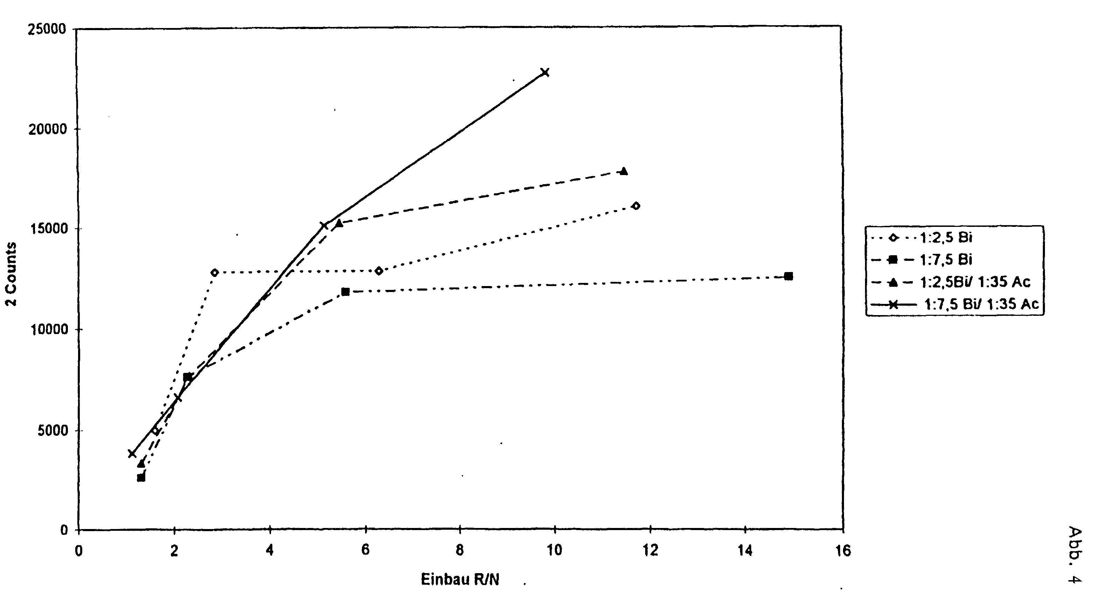

- Abb. 3 und 4: die Größe des Elektrochemilumineszenz-Meßsignals bei derivatisierten und nichtderivatisierten Rutheniummarkierten Antikörpern in einem heterogenen und einem homogenen Testsystem.

### Beispiele

### 1. Herstellung von Reagenzien

Konjugate aus polyklonalen Schaf-Anti-T4-lgG-Antikörpern und Biotin sowie Ruthenium(bipyridyl)₃-Komplex (Rubpy) wurden in verschiedenen Biotinylierungs- und Ruthenylierungsgraden hergestellt.

### Biotinylierung:

Immunglobulin (IGG) wurde in 0,1 M Kaliumphosphat, pH 8,4 zu einer Konzentration von 10 mg/ml gelöst. Es wurde pro 1 ml Lösung die für die gewählte Stöchiometrie nötige molare Menge Biotin-∈-amino-capronsäure-N-hydroxysuccinimidester gelöst in 50 µl wasserfreiem Dimethylsulfoxid zugesetzt. Nach 90 min Rühren bei 25°C wurde die Reaktion durch Zugabe von 10 µmol Lysin pro 1 ml Ansatz gestoppt. Dann wurde bei 4°C gegen 25 mM Kaliumphosphat/50 mM NaCl, pH 7,0 dialysiert und das jeweilige biotinylierte IGG (IGG-BI) lyophilisiert.

### Ruthenylierung:

Das lyophilisierte IGG-BI (mit dem jeweils gewählten Biotinylierungsgrad) wurde in 0,15 M Kaliumphosphat/0,15 M NaCl, pH 7,8 zu 10 mg/ml gelöst. Pro 1 ml Lösung wurde die für die gewählte Stöchiometrie nötige molare Menge von Ruthenium(2,2'-bipyridyl)₂(4-(3-(N-hydroxysuccinimidylcarboxy)propyl]-4'-methyl-2,2'-bipyridin, gelöst in 50 µl wasserfreiem Dimethylsulfoxid, zugesetzt und 90 min bei 25°C gerührt.

Für die Konjugatvarianten ohne Acetylierung wurde die Reaktion direkt durch Zugabe von 10 µmol Lysin pro 1 ml Lösung gestoppt. Für die acetylierten Konjugatvarianten wurde pro 1 ml Lösung 2,35 µmol Acetyl-N-hydrocysuccinimidester (gelöst in 50 ml wasserfreiem Dimethylsulfoxid) hinzugefügt (Molverhältnis Acetylierungsreagenz pro IGG = 35:1) und weitere 45 min bei 25°C gerührt. Dann erst wurde mit Lysin wie oben angegeben gestoppt.

Beide Konjugattypen wurden gegen 25 mM Kaliumphosphat/0,15 M NaCl, pH 7,0 dialysiert und dann lyophilisiert.

Es wurden Chargen mit einem Biotinylierungsgrad von 1:2,5 und einem Ruthenylierungsgrad von 1:2,5 (A), 1:5 (B), 1:12 (C) und 1:25 (D) sowie mit einem Biotinylierungsgrad von 1:7,5 und einem Ruthenylierungsgrad von 1:2,5 (E), 1:5 (F), 1:12 (G) und 1:25 (H) hergestellt. Die entsprechenden acetylierten Chargen sind mit der Bezeichnung "ac" versehen.

Die Analysedaten dieser Antikörperchargen sind in der nachfolgenden Tabelle 1 dargestellt:

**Tabelle 1**

| Biotinylierungsgrad 1:2,5 | | | | | |
|---|---|---|---|---|---|
| **Charge Nr.** | **ml** | **ε455 nm** | **Prot. BCA** | **Einbau R/N** | **Σ Protein** |
| A 1:2,5 | 5,3 | 1,30 | 8,9 | 1,6 | 47 mg |
| B 1:5 | 4,3 | 2,40 | 9,2 | 2,86 | 39,6 mg |
| C 1:12 | 4,5 | 5,20 | 9,06 | 6,28 | 40,8 mg |
| C 1:25 | 4,0 | 8,16 | 7,6 | 11,72 | 30,5 mg |

| Biotinylierungsgrad 1:7,5 | | | | | |
|---|---|---|---|---|---|
| **Charge Nr.** | **ml** | **ε455 nm** | **Prot. BCA** | **Einbau R/N** | **Σ Protein** |
| E 1:2,5 | 4,5 | 1,14 | 9,48 | 1,32 | 42,7 mg |
| F 1:5 | 4,6 | 1,92 | 9,24 | 2,28 | 42,5 mg |
| G 1:12 | 4,3 | 4,86 | 9,54 | 5,58 | 41 mg |
| H 1:25 | 4,1 | 5,62 | 6,0 | 14,89 | 24,6 mg |

| Biotinylierungsgrad 1:2,5; Acetylierungsgrad 1:35 | | | | | |
|---|---|---|---|---|---|
| **Charge Nr.** | **ml** | **ε455 nm** | **Prot. BCA** | **Einbau R/N** | **Σ Protein** |
| Aac 1:2,5 | 3,9 | 1,06 | 8,88 | 1,31 | 34,6 mg |
| Bac 1:5 | 4,1 | 1,97 | 9,3 | 2,32 | 38,1 mg |
| Cac 1:12 | 2,27 | 4,56 | 9,18 | 5,44 | 20,8 mg |
| Dac 1:25 | 3,9 | 7,86 | 7,5 | 11,47 | 29,3 mg |

| Biotinylierungsgrad 1:7,5; Acetylierungsgrad 1:35 | | | | | |
|---|---|---|---|---|---|
| **Charge Nr.** | **ml** | **ε455 nm** | **Prot. BCA** | **Einbau R/N** | **Σ Protein** |
| Eac 1:2,5 | 4,1 | 0,96 | 9,36 | 1,12 | 38,4 mg |
| Fac 1:5 | 4,0 | 2,01 | 10,56 | 2,08 | 42,2 mg |
| Gac 1:12 | 3,7 | 5,00 | 10,68 | 5,13 | 39,5 mg |
| Hac 1:25 | 4,0 | 5,88 | 6,54 | 9,847 | 26,2 mg |

Die Gesamtmenge Protein im Ansatz (Σ Protein) wurde bestimmt mit dem BCA-Reagenz von Pierce nach Herstellerangaben.

R/N bedeutet durchschnittliche Zahl von Rutheniumkomplexen pro 1 IGG Molekül.

R bedeutet die Menge (nmol) Ruthenium-Komplex in 1 ml Lösung und errechnet sich aus der in der Tabelle angegebenen Extinktion (∈455 nm) durch Division mit dem Extinktionskoeffizienten 13,7 für den Komplex.

N bedeutet die Menge (nmol) IGG in 1 ml Lösung und errechnet sich aus dem in der Tabelle angegebenen Wert Prot. BCA (mg IGG-Protein pro 1 ml Lösung) durch Division mit dem Molekulargewichtsfaktor 150 für IGG.

### 2. Cyclovoltammetriemessung der Antikörperkonjugate

Die Meßbedingungen waren wie folgt: Arbeitselektrode: Glas-Carbon mit einem Durchmesser von 3 mm; Gegenelektrode: Pt; Bezugselektrode: Ag/AgCl in 3 M KCI; 100 mV/s; Raumtemperatur.

5 mg des Antikörperkonjugats wurden in 1 ml Phosphatpuffer (0,1 M KH₂PO₄ + 0,1 M KOH, pH 7) gelöst.

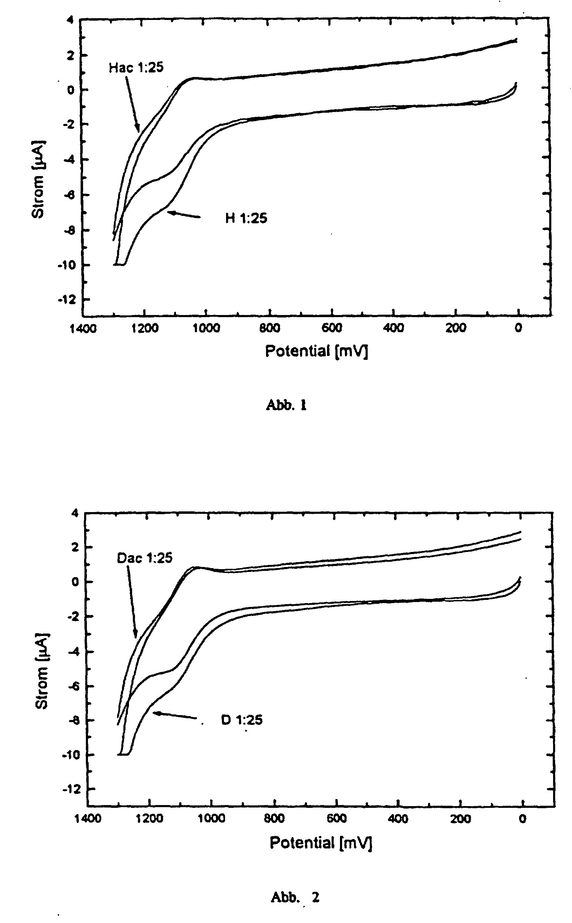

Der Vergleich zwischen den acetylierten Konjugaten (Hac und Dac) mit den entsprechenden nichtacetylierten Konjugaten (H und D) ist in den Abbildungen 1 und 2 gezeigt. Dort ist zu erkennen, daß der Oxidationsstrom nach Acetylierung abnimmt, d.h. der katalytische Effekt durch oxidierbare Gruppen, z.B. freie Aminogruppen, wird durch die Acetylierung zurückgedrängt. Der Reduktionspeak ist in Abbildung 2 etwas stärker ausgeprägt.

Aus diesen Daten ist ersichtlich, daß in einem Elektrochemilumineszenz-Nachweisverfahren eine partielle Oxidation freier Aminogruppen in biologischen Substanzen durch das Metallkation, z.B. Ruthenium (III), erfolgt und daß dieses Verhalten durch eine Derivatisierung mittels Acetylierung wieder aufgehoben werden kann.

### 3. Einfluß der Acetylierung auf das Meßsignal

Die Untersuchung wurde mit den in Beispiel 1 hergestellten Konjugaten in einem homogenen Test und in einem heterogenen Test (Anwesenheit von Streptavidin-beschichteten Mikrobeads) an einem Elecsys®2010 Seriengerät durchgeführt.

### Testführung:

Die Konjugate in Tabelle 1 wurden in einer Konzentration von 125 ng/ml in Elecsys® ProCell (0, 3 M Phosphatpuffer + 0,18 M Tripropylamin + 0,1 % Detergenz, pH 6,8) gelöst.

Diese Lösungen wurden im homogenen Test unverdünnt eingesetzt und gemessen.

Im heterogenen Test wurden 15 µl der Konjugatlösungen mit 185 µl einer Beadsuspension von 136 µg Streptavidin-beschichteten Beads/ml 9 min bei 37°C inkubiert. Nach einer Bound-free-Trennung wurden die Beads gemessen.

Beide Applikationen wurden am Elecsys® 2010 Gerät durchgeführt.

Als Ergebnis dieser Untersuchungen wurde festgestellt, daß insbesondere bei den hochbiotinylierten Konjugaten (E bis H) aufgrund der Acetylierung eine signifikante Erhöhung des Meßsignals gefunden wird. Bei den niedrigbiotinylierten Konjugaten (A bis D) ist diese Wirkung schwächer ausgeprägt.

Weiterhin wurde gefunden, daß ein nichtacetyliertes Konjugat sowohl in homogenen als auch im heterogenen Test ab einem Ruthenylierungsgrad > 1:12 kein höheres Meßsignal mehr liefert. Durch Capping konnte eine signifikante Erhöhung des Meßsignals erzielt werden.

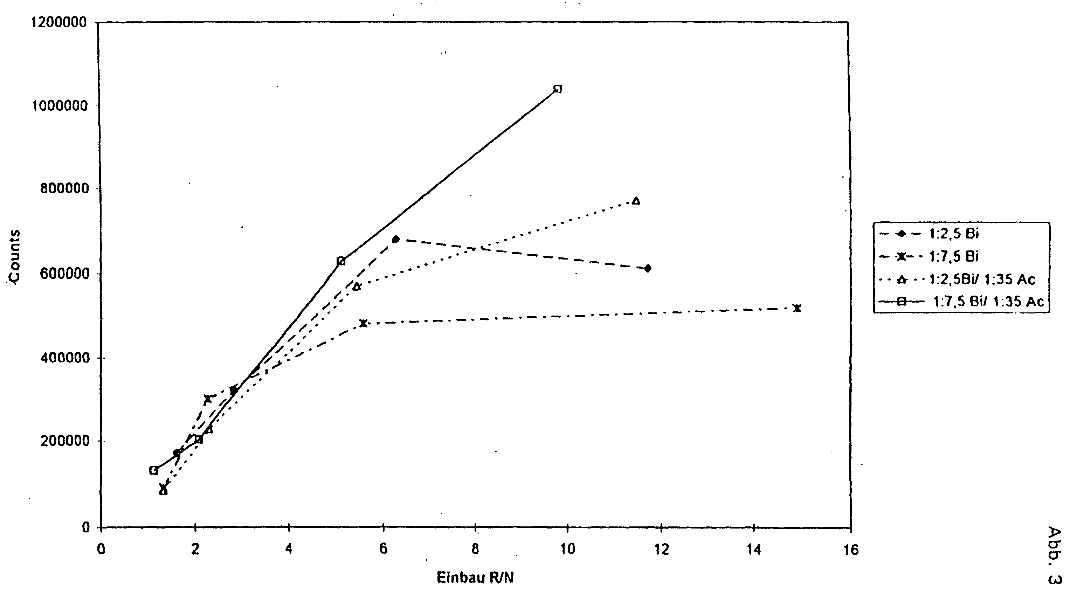

Das Ergebnis dieser Untersuchungen ist in den Abbildungen 3 (heterogener Test) und 4 (homogener Test) zusammengefaßt.

## Patentansprüche

1. Verfahren zum Nachweis eines Analyten in einer Probe durch Chemilumineszenz oder Elektrochemilumineszenz, wobei man die Probe mit einem Testreagenz in Kontakt bringt, das mindestens eine Chemilumineszenz- oder Elektrochemilumineszenz-Markierungsgruppe gekoppelt an einen analytspezifischen Rezeptor und weitere Testkomponenten enthält,
**dadurch gekennzeichnet,**
**daß** oxidierbare chemische Gruppen auf dem markierten Rezeptor oder/und weiteren Testkomponenten durch Derivatisierung geschützt werden.

2. Verfahren nach Anspruch 1,
**dadurch gekennzeichnet,**
**daß** die oxidierbaren chemischen Gruppen ausgewählt werden aus primären Aminogruppen, sekundären Aminogruppen, Schiffbasen, Thiolgruppen, Acetalgruppen und Ketalgruppen.

3. Verfahren nach einem der vorhergehenden Ansprüche,
**dadurch gekennzeichnet,**
**daß** die Derivatisierung durch eine Acylierung erfolgt.

4. Verfahren nach einem der vorhergehenden Ansprüche,
**dadurch gekennzeichnet,**
**daß** die derivatisierten Substanzen ausgewählt werden aus Peptiden, Polypeptiden, Nukleinsäuren, Nukleinsäureanaloga, Zuckern und Haptenen.

5. Verfahren nach einem der vorhergehenden Ansprüche,
**dadurch gekennzeichnet,**
**daß** die Markierungsgruppen ausgewählt werden aus Metallkomplexen mit einer Struktur der allgemeinen Formel (I):
Mₙ(L₁L₂L₃)ₘ (I)
worin
M ein zwei- oder dreiwertiges Metallkation ausgewählt aus Seltenerde- und Übergangsmetallkationen ist,
L₁, L₂ und L₃ gleich oder verschieden sind und Liganden mit mindestens 2 stickstoffhaltigen Heterocyclen sind und
n und m jeweils unabhängig ganze Zahlen von 1 bis 10 sind.

6. Verfahren nach einem der vorhergehenden Ansprüche,
**dadurch gekennzeichnet,**
**daß** es als heterogener Test durchgeführt wird, wobei man die Markierung an einer Festphase immobilisiert und das Vorhandensein oder/und die Menge des Analyten über die an der Festphase immobilisierte Markierung bestimmt.

7. Verfahren nach einem der vorhergehenden Ansprüche,
**dadurch gekennzeichnet,**
**daß** ein nichtmarkierter analytspezifischer Rezeptor in derivatisierter Form eingesetzt wird.

8. Verfahren nach einem der vorhergehenden Ansprüche,
**dadurch gekennzeichnet,**
**daß** eine Festphasenbeschichtung in derivatisierter Form eingesetzt wird.

9. Verfahren nach einem der vorhergehenden Ansprüche,
**dadurch gekennzeichnet,**
**daß** ein Entstörungsreagenz in derivatisierter Form eingesetzt wird.

10. Reagenzienkit zum Nachweis eines Analyten, umfassend
(a) einen analytspezifischen Rezeptor, der mindestens eine Chemilumineszenz- oder Elektrochemilumineszenz-Markierungsgruppe trägt, und
(b) weitere Testkomponenten,
**dadurch gekennzeichnet,**
**daß** oxidierbare chemische Gruppen auf dem markierten Rezeptor oder/und weiteren Testkomponenten durch Derivatisierung geschützt sind.

11. Verwendung eines Reagenzienkits nach Anspruch 10 in einem Verfahren zum Nachweis eines Analyten.

12. Konjugat einer biologischen Substanz und mindestens einer Chemilumineszenz- oder Elektrochemilumineszenz-Markierungsgruppe,
**dadurch gekennzeichnet,**
**daß** oxidierbare chemische Gruppen auf der biologischen Substanz durch Derivatisierung geschützt sind.

## Claims

1. Method for the detection of an analyte in a sample by chemiluminescence or electrochemiluminescence in which the sample is contacted with a test reagent which contains at least one chemiluminescent or electrochemiluminescent labelling group coupled to an analyte-specific receptor and other test components,
**characterized in that**
the oxidizable chemical groups on the labelled receptor or/and on other test components are protected by derivatization.

2. Method as claimed in claim 1,
**characterized in that**
the oxidizable chemical groups are selected from primary amino groups, secondary amino groups, Schiff's bases, thiol groups, acetal groups and ketal groups.

3. Method as claimed in one of the previous claims,
**characterized in that**
the derivatization is carried out by acylation.

4. Method as claimed in one of the previous claims,
**characterized in that**
the derivatized substances are selected from peptides, polypeptides, nucleic acids, nucleic acid analogues, sugars and haptens.

5. Method as claimed in one of the previous claims,
**characterized in that**
the labelling groups are selected from metal complexes having a structure of the general formula (I):
Mₙ(L₁L₂L₃)ₘ (I)
in which
M is a divalent or trivalent metal cation selected from rare-earth and transition metal cations,
L₁, L₂ and L₃ are the same or different and are ligands with at least two heterocycles containing nitrogen and
n and m are each independently integers from 1 to 10.

6. Method as claimed in one of the previous claims,
**characterized in that**
it is carried out as a heterogeneous test in which the label is immobilized on a solid phase and the presence or/and the amount of the analyte is determined by means of the label immobilized on the solid phase.

7. Method as claimed in one of the previous claims,
**characterized in that**
a non-labelled analyte-specific receptor in a derivatized form is used.

8. Method as claimed in one of the previous claims,
**characterized in that**
a solid phase coating in a derivatized form is used.

9. Method as claimed in one of the previous claims,
**characterized in that**
an interference-eliminating reagent in a derivatized form is used.

10. Reagent kit for detecting an analyte comprising,
(a) an analyte-specific receptor which carries at least one chemiluminescent or electrochemiluminescent labelling group and
(b) other test components
**characterized in that**
the oxidizable chemical groups on the labelled receptor or/and on other test components are protected by derivatization.

11. Use of a reagent kit as claimed in claim 10 in a method for detecting an analyte.

12. Conjugate of a biological substance and at least one chemiluminescent or electrochemiluminescent labelling group,
**characterized in that**
the oxidizable chemical groups on the biological substance are protected by derivatization.

## Revendications

1. Procédé de détection d'un analyte dans un échantillon par chimioluminescence ou électrochimioluminescence, dans lequel on met l'échantillon en contact avec un réactif de test qui contient au moins un groupe de marquage par chimioluminescence ou par électrochimioluminescence couplé à un récepteur spécifique de l'analyte et d'autres composants de test,
**caractérisé en ce que**,
des groupes chimiques oxydables se trouvant sur le récepteur marqué et/ou d'autres composants de test sont protégés par dérivation.

2. Procédé selon la revendication 1,
**caractérisé en ce que** les groupes chimiques oxydables sont choisis parmi des groupes amino primaires, des groupes amino secondaires, des bases de Schiff, des groupes thiol, des groupes acétal et des groupes cétal.

3. Procédé selon l'un quelconque des revendications précédentes,
**caractérisé en ce que** la dérivation est réalisée par une acylation.

4. Procédé selon l'une quelconque des revendications précédentes,
**caractérisé en ce que** les substances dérivées sont choisies parmi des peptides, des polypeptides, des acides nucléiques, des analogues d'acide nucléique, des sucres et des haptènes.

5. Procédé selon l'une quelconque des revendications précédentes,
**caractérisé en ce que** les groupes de marquage sont choisis parmi des complexes métalliques présentant une structure de formule générale (I) :
Mₙ(L₁L₂L₃)ₘ (I)
dans laquelle
M est un cation métallique bivalent ou trivalent choisi parmi des cations des terres rares et des cations de métaux de transition,
L₁, L₂, et L₃ sont identiques ou différents et sont des ligands avec au moins deux hétérocycles azotés, et
n et m sont chacun indépendamment des nombres entiers de 1 à 10.

6. Procédé selon l'une quelconque des revendications précédentes,
**caractérisé en ce qu'**il est mis en oeuvre comme test hétérogène, où l'on immobilise le marquage à une phase solide, et l'on détermine la présence et/ou la quantité de l'analyte via le marquage immobilisé à la phase solide.

7. Procédé selon l'une quelconque des revendications précédentes,
**caractérisé en ce que** l'on utilise un récepteur non marqué et spécifique de l'analyte sous forme dérivée.

8. Procédé selon l'une quelconque des revendications précédentes,
**caractérisé en ce que** l'on utilise un revêtement de phase solide sous forme dérivée.

9. Procédé selon l'une quelconque des revendications précédentes,
**caractérisé en ce que** l'on utilise un réactif d'antiparasitage sous forme dérivée.

10. Kit de réactifs pour la détection d'analyte, comprenant :
(a) un récepteur spécifique à l'analyte, qui porte au moins un groupe de marquage par chimioluminescence ou électrochimioluminescence, et
(b) d'autres composants de test,
**caractérisé en ce que** des groupes chimiques oxydables se trouvant sur le récepteur marqué et/ou d'autres composants de test sont protégés par dérivation.

11. Utilisation d'un kit de réactifs selon la revendication 10 dans un procédé de détection d'analyte.

12. Conjugué d'une substance biologique et d'au moins un groupe de marquage par chimioluminescence ou électrochimioluminescence,
**caractérisé en ce que** des groupes chimiques oxydables se trouvant sur la substance biologique sont protégés par dérivation.
